# EUROPÄISCHE PATENTSCHRIFT

(11) **EP 0 301 198 B1**
(45) Veröffentlichungstag und Bekanntmachung des Hinweises auf die Patenterteilung: **07.07.1993**
(21) Anmeldenummer: 88108667.2
(22) Anmeldetag: 31.05.1988
(51) Int. Cl.: A61B 17/22, A61B 6/10

(54) **Medizintechnische Sicherheitseinrichtung für Diagnose und Therapie, insbesondere für die berührungsfreie Lithotripsie**
Medical safety device for diagnosis and therapy, in particular for contact-free lithotripsy
Dispositif de sécurité médical pour le diagnostique et la thérapie, en particulier pour la lithotripsie sans contact

(30) Priorität: 29.07.1987 DE 3725129
(43) Veröffentlichungstag der Anmeldung: 01.02.1989
(73) Patentinhaber: DORNIER MEDIZINTECHNIK GMBH, D-82101 Germering (DE)
(72) Erfinder: Erhardt, Wolfgang, Dipl.-Ing., D-8080 Fürstenfeldbruck (DE); Grözinger Reiner, Dipl.-Ing., D-8031 Alling (DE); Wess, Othmar, Dr. Dipl.-Phys, D-8000 München (DE)
(74) Vertreter: Landsmann, Ralf, Dipl.-Ing.

(56) Entgegenhaltungen:
- EP-A- 0 273 256
- DE-A- 3 343 924
- JP-A-57 151 938
- US-A- 4 610 249

## Beschreibung

Die Erfindung betrifft eine medizinische Sicherheitseinrichtung für Diagnose und Therapie, insbesondere für die berührungsfreie Lithotripsie.

Eine Vorrichtung zur berührungsfreien Lithotripsie ist beispielsweise aus der **US-A-4 610 249** bekannt.

Bei Diagnose und Therapie befindet sich der Körper des Patienten auf einer Patientenliege und das Diagnose- oder Behandlungsgerät wird vom Arzt oder Klinikpersonal manuell oder motorisch an den Stellen positioniert, an denen die Diagnose oder Behandlung erfolgen soll. Bei der motorischen Positionierung ist darauf zu achten, daß der Anpreßdruck nicht zu Veränderungen im Körper des Patienten, Verletzungen oder zu Schmerzen führt. Durch vorsichtige Handlungsweise können Probleme bei nicht-narkotisierten Patienten vermieden werden.

**JP-A- 57 151 938** offenbart in der Zusammenfassung und Figur eine Sicherheitseinrichtung zur Vermeidung von Berührung zwischen einer Szintillationskamera und einem Patienten, der auf einer Patientenliege gelagert sein muß, obwohl das in der Literaturstelle nicht ausdrücklich erwähnt ist. Hierzu ist der Abtaster der Szintillationskamera mit einem Luftkissen aus einem flexiblen Material ausgestattet, das den Abtaster in Richtung auf den Körper des Patienten überragt. Bei Kontakt zwischen dem Luftkissen und dem Patienten wird mittels eines Drucksensors und nachgeschalteten Flipflop die Bewegung des Abtasters unterbrochen.

Der Erfindung liegt die Aufgabe zugrunde, bei der berührungsfreien Lithotripsie eine Sicherheitseinrichtung so anzuordnen, dass einerseits der Körper eines Patienten optimal angekoppelt ist und andererseits der Patient nicht verletzt werden kann, wenn bei der Ortung des Konkrements durch den behandelnden Arzt die Patientenliege so positioniert wird, dass das zu zertrümmernde Konkrement genau in den zweiten Brennpunkt bezüglich der Stosswellenquelle zu liegen kommt.

Diese Aufgabe wird durch den Gegenstand des einzigen Anspruchs gelöst.

Die Erfindung wird nachfolgend näher erläutert.

Es zeigen:

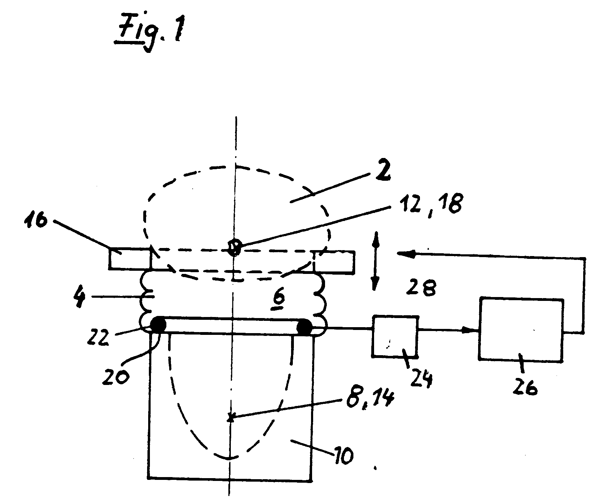

- Fig. 1: eine Einrichtung der erfindungsgemässen Art in prinzipieller Darstellung und
- Fig. 2: die Integration einer erfindungsgemässen Sicherheitseinrichtung in ein Behandlungsgerät.

Bei Stosswellenapplikationen zur Zertrümmerung von Konkrementen in Organen des menschlichen Körpers erfolgt die Einkopplung der Stosswellen in den Körper **2** des Patienten mit Kissen **4**, die mit einem geeigneten Koppelmedium **6** (z.B. Wasser) gefüllt sind. Die Stosswellen werden dabei in einem Brennpunkt **8** eines Rotationsellipsoids **10** erzeugt und im zweiten Brennpunkt **12** innerhalb des Körpers des Patienten fokussiert. In diesem Brennpunkt befindet sich das Konkrement.

Es ist hierzu erforderlich, den Körper **2** des Patienten in bezug auf die Stosswellenquelle **14** mit Hilfe einer Patientenliege **16** so zu positionieren, dass das ein zu zertrümmerndes Konkrement **18** genau in den zweiten Brennpunkt **12** bezüglich der Stosswellenquelle **14** zu liegen kommt. Die Patientenliege **16** weist eine Ausnehmung auf, sodass ein Teil des Körpers des Patienten gegenüber der Stosswellenquelle **14** frei liegt.

In Abhängigkeit von der Korpulenz des Patienten kann bei dicken Patienten der Rand **20** des Ellipsoids **10** mit dem Körper **2** des Patienten kollidieren.

Um dies zu vermeiden ist auf dem Rand **20** ein ringförmiger Schlauch **22** angeordnet, der mit einem Fluid oder Gas gefüllt ist. Bei der Annäherung des Körpers des Patienten an den Rand **20** des Ellipsoids **10** wird bei Berührung des Schlauches **22** der Druck im Fluid erhöht, mittels eines Druckschalters **24** detektiert und mittels einer Elektronik **26** ausgewertet.

Als Druckschalter **24** wird ein Differenzdrucksensor verwendet, der beim Überschreiten oder Unterschreiten eines voreingestellten Druckgrenzwerts eine Schaltfunktion ausführt.

Mit dem Ausgangssignal des Drucksensors wird die Kollisionsbewegung der Patientenliege **16** gestoppt; eine Bewegung der Liege ist dann nur noch in von Stosswellenquelle **14** abgewandter Richtung möglich.

Der Druck im Fluid innerhalb des Schlauchs **22** wird gegenüber dem barometrischen Luftdruck als Bezugsdruck gemessen. Diese Verfahrensweise verhindert eine fehlerhafte Messung bei Luftdruckänderungen der Atmosphäre, wenn die Ansprechempfindlichkeit im hPa (hektopascal) Bereich liegt. Druckschwankungen des Koppelmediums **6** innerhalb des Kissens **4** liegen unterhalb der Ansprechschwelle der aus Schlauch **22,** Drucksensor **24** und Elektronik **26** bestehenden Sicherheitseinrichtung **28**.

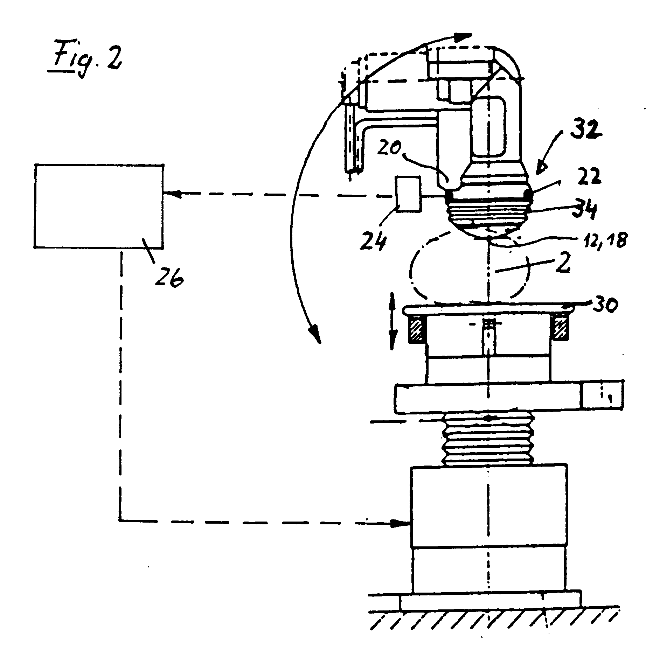

Wie in Fig. 2 gezeigt, ist die Sicherheitseinrichtung universell für unterschiedliche Ankoppelsysteme geeignet. In Fig. 2 befindet sich der Körper **2** des Patienten auf einer motorisch verschiebbaren Liege **30** und eine Behandlungseinrichtung **32** ist auf der gegenüberliegenden Seite angeordnet. Die Ankoppelung erfolgt mittels eines Kissens **34** und die Sicherheitseinrichtung **28** befindet sich vor den starren Bauteilen **20** des Behandlungsgeräts, sodass es zu keinen Kollisionen mit dem Körper kommen kann, wenn die elastische Nachgiebigkeit des Kissens **34** erschöpft ist.

## Patentansprüche

1. Sicherheitseinrichtung zur Vermeidung der mechanischen Kollision von Körperteilen eines Patienten, der zur Durchführung einer berührungsfreien Lithotripsie auf einer Patientenliege (**16, 30**) gelagert ist, mit peripheren starren Bauteilen (**20**) eines Behandlungsgeräts (**32**), das ein Teil eines Rotationsellipsoids (**10**) ist, wobei die Patientenliege (**16**) und das Behandlungsgerät (**32**) relativ zueinander in Behandlungsposition bewegbar sind und ein elastisch verformbarer Schlauch (**22**) vorhanden ist, der mit einer Flüssigkeit oder einem Gas gefüllt ist, am Rand (**20**) des Ellipsoids (**10**) befestigt ist und sich innerhalb eines flüssigkeitsgefüllten Koppelkissens (**4, 34**) befindet, das die Verbindung zwischen dem Behandlungsgerät und dem Körper (**2**) des Patienten bildet und mindestens einen Drucksensor im oder am elastischen Schlauch, der einen Schalter (**24**) zur Unterbrechung der Bewegung der Patientenliege (**16**) betätigt, wenn der Schlauch (**22**) mit dem Körper (**2**) des Patienten in Berührungskontakt kommt.

## Claims

1. Safety device for avoiding the mechanical collision of parts of the body of a patient lying on a couch (16, 30) for the performance of a contact-free lithotripsy, and having peripheral rigid components (20) of a treatment apparatus (32) which is part of an ellipsoid of revolution (10), wherein the couch (16) and the treatment apparatus (32) can be moved relative to one another in the treatment position and a resiliently deformable hose (22) is present which is filled with a liquid or a gas, is secured at the edge (20) of the ellipsoid (10), and is located within a fluid-filled coupling cushion (34) providing the connection between the treatment apparatus and the patient's body (2), and in or on the resilient hose at least one pressure sensor which activates a switch (24) for interrupting the movement of the couch (16) when the hose (22) comes into contact with the patient's body (2).

## Revendications

1. Dispositif de sécurité pour éviter la collision mécanique de parties du corps d'un patient, qui repose sur une couchette (16, 30) en vue d'effectuer une lithotriptie sans contact, avec des éléments périphériques rigides (20) d'un appareil de traitement (32) qui fait partie d'un ellipsoïde de révolution (10), la couchette (16) et l'appareil de traitement (32) étant mobiles l'un par rapport à l'autre en position de traitement, et un tuyau souple élastiquement déformable (22) étant présent, qui est rempli d'un liquide ou d'un gaz, est fixé sur le bord de l'ellipsoïde (10), se trouve à l'intérieur d'un coussin d'accouplement (34) rempli de liquide et réalisant la liaison entre l'appareil de traitement et le corps (2) du patient, et un capteur de pression, étant présent dans ou sur le tuyau souple, capteur qui actionne un commutateur (24) pour interrompre le mouvement de la couchette (16) lorsque le tuyau souple (22) arrive en contact avec le corps (2) du patient.
